(19) Europäisches Patentamt
European Patent Office
Office européen des brevets

(11) **EP 3 645 692 B1**

(12) **EUROPEAN PATENT SPECIFICATION**

(45) Date of publication and mention of the grant of the patent:
**31.12.2025 Bulletin 2026/01**

(21) Application number: **18734801.6**

(22) Date of filing: **27.06.2018**

(51) International Patent Classification (IPC):
***C11D 3/386*** *(2006.01)* ***C11D 3/22*** *(2006.01)*
***C11D 3/04*** *(2006.01)* ***C11D 17/00*** *(2006.01)*
***C11D 3/20*** *(2006.01)*

(52) Cooperative Patent Classification (CPC):
**C11D 3/38672; C11D 3/046; C11D 3/048; C11D 3/2075; C11D 3/222; C11D 3/386; C11D 11/0082; C11D 17/0013**

(86) International application number:
**PCT/EP2018/067227**

(87) International publication number:
**WO 2019/002356 (03.01.2019 Gazette 2019/01)**

(54) **ENZYME SLURRY COMPOSITION**

ENZYMSCHLAMMZUSAMMENSETZUNG

COMPOSITION DE SUSPENSION ENZYMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2017 PCT/CN2017/091000**

(43) Date of publication of application:
**06.05.2020 Bulletin 2020/19**

(73) Proprietor: **Novozymes A/S**
**2880 Bagsvaerd (DK)**

(72) Inventors:
- **WEI, Wei**
  **Beijing 100085 (CN)**
- **LI, Zhenwei**
  **Beijing**
  **Haidian District 100085 (CN)**
- **YU, Jia**
  **Beijing**
  **Haidian District 100085 (CN)**
- **SIMONSEN, Ole**
  **2880 Bagsvaerd (DK)**
- **ANDERSEN, Kim, Bruno**
  **2880 Bagsvaerd (DK)**
- **JACOBSEN, Carsten**
  **2880 Bagsvaerd (DK)**
- **XU, Yuan**
  **Beijing**
  **Haidian District 100085 (CN)**
- **YANG, Zaizhou**
  **Beijing**
  **Haidian District 100085 (CN)**

(74) Representative: **NVS EPO Representatives**
**Krogshoejvej 36**
**2880 Bagsvaerd (DK)**

(56) References cited:
**EP-A1- 0 506 791** **EP-A2- 0 450 702**
**EP-B1- 0 506 791** **WO-A1-2009/102854**
**WO-A1-2016/201040** **CA-A1- 2 297 443**
**CA-A1- 2 300 630** **CA-A1- 2 850 079**
**US-A1- 2009 156 454**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 645 692 B1

## Description

### FIELD OF THE INVENTION

The present invention relates to an enzyme slurry composition, which exhibits excellent stability and reduced production cost.

### BACKGROUND

Detergent compositions often comprise enzymes alongside other components such as surfactants. A variety of different enzymes may be used in such detergent compositions in order to help clean substrates, such as laundry or dishware. The enzymes may include proteases to break down proteinaceous material, lipases to break down fatty materials, and amylases to break down carbohydrate-based material.

Enzymes are often added in liquid form as a solution or in solid form as a granulate during the preparation of the detergent composition. Granulates can contain high amounts of active enzyme, while the concentration (enzyme activity) of liquid enzyme products suffers from limitations in the enzyme solubility. At the same time, liquid enzyme products require expensive formulation ingredients to retain a reasonable enzyme stability.

EP 450 702 A2 discloses a process for preparing liquid enzymatic detergent compositions by adding the enzyme in the form of a slurry of the enzyme in liquid nonionic surfactant.

### SUMMARY OF THE INVENTION

In a first aspect, the present invention provides an enzyme slurry composition comprising

(a) enzyme particles;
(b) a water-soluble salt selected from the group consisting of sodium sulfate, potassium sulfate, magnesium sulfate, zinc sulfate, ammonium sulfate, sodium nitrate, potassium nitrate, magnesium nitrate, zinc nitrate, ammonium nitrate, sodium chloride, potassium chloride, magnesium chloride, zinc chloride, ammonium chloride, sodium acetate, potassium acetate, magnesium acetate, zinc acetate, ammonium acetate, sodium citrate, potassium citrate, magnesium citrate, zinc citrate, ammonium citrate, sodium formate, potassium formate, magnesium formate, zinc formate, and ammonium formate;
(c) at least 0.1% w/w of xanthan gum, guar gum, locust bean gum, welan gum, gellan gum, alginate, carrageenan, or microcrystalline cellulose; and
(d) at least 50% w/w of water;

wherein the enzyme particles consist of enzyme crystals or a spray dried enzyme composition, and wherein the enzyme slurry comprises at least 1% w/w of active enzyme protein.

Other aspects and embodiments of the invention are apparent from the description and examples.

### DETAILED DESCRIPTION

The aqueous enzyme slurry of the invention is a product format that has enzyme particles suspended in an aqueous stabilizing solution. A viscosity regulating agent is added to retain a uniform distribution of the enzyme particles in the slurry, even after extended storage.

The enzyme slurry dissolves quickly after being added into a detergent manufacturing process, and therefore it can be used without influencing a detergent production procedure at a customer. The dissolution time of the enzyme slurry in the detergent formulation can be reduced even further by adding enzyme inhibitors.

The enzymatic stability of the enzyme slurry is excellent compared to traditional liquid product formats. Further, the relative amount of active enzyme can be higher than in traditional liquid product formats, and therefore the production costs per unit of enzyme activity are lower. Since the amount of water in the enzyme slurry is usually about 60-80% w/w, the cost of formulation ingredients is low.

#### Enzyme particles

The enzyme slurry of the invention comprises enzyme particles. The enzyme particles may contain one or more of the enzymes described below. Different enzymes can be mixed or separated (such as arranged in layers) in the enzyme particle.

The enzyme particles consist of enzyme crystals or a spray dried enzyme composition. Typically, the particle size,

measured as equivalent spherical diameter (volume based average particle size), of the enzyme particles is below 2 mm, preferably below 1 mm, below 0.5 mm, below 0.25 mm, or below 0.1 mm; and above 0.05 μm, preferably above 0.1 μm, above 0.5 μm, above 1 μm, above 5 μm or above 10 μm.

In a preferred embodiment, the particle size of the enzyme particles is from 0.5 μm to 100 μm.

The enzyme particles contain at least 1% w/w enzyme protein, preferably at least 5% w/w enzyme protein, at least 10% w/w enzyme protein, at least 20% w/w enzyme protein, at least 30% w/w enzyme protein, at least 40% w/w enzyme protein, at least 50% w/w enzyme protein, at least 60% w/w enzyme protein, at least 70% w/w enzyme protein, at least 80% w/w enzyme protein, or at least 90% w/w enzyme protein.

Enzyme crystallization may be carried out in a number of ways, as known in the art (e.g., as described in WO 91/09943 or WO 94/22903).

The enzyme may be formulated in the enzyme particle as known in the art for solid enzyme formulations, such as formulations for reducing dust, improving stability and/or modifying release rate of the enzyme. The enzyme particle may also be formulated in a matrix or coated with agents suppressing dissolution of the enzyme particle in the PVOH/film solution used for preparing the water-soluble film.

The enzyme molecules on the surface of the enzyme particles may also be cross-linked, like CLECs (Cross-Linked Enzyme Crystals) or CLEA (Cross-Linked Enzyme Aggregate).

**Water-soluble salt**

One or more water-soluble salt(s) is/are added to the (aqueous) enzyme slurry composition to avoid dissolution of the enzyme particles in the aqueous slurry after storage. The water-soluble salt is selected from the group consisting of sodium sulfate, potassium sulfate, magnesium sulfate, zinc sulfate, ammonium sulfate, sodium nitrate, potassium nitrate, magnesium nitrate, zinc nitrate, ammonium nitrate, sodium chloride, potassium chloride, magnesium chloride, zinc chloride, ammonium chloride, sodium acetate, potassium acetate, magnesium acetate, zinc acetate, ammonium acetate, sodium citrate, potassium citrate, magnesium citrate, zinc citrate, ammonium citrate, sodium formate, potassium formate, magnesium formate, zinc formate, and ammonium formate.

The water-soluble salt is added to the enzyme slurry in amount sufficient to avoid dissolution of the enzyme particles after storage. This is easy to measure because the residual enzyme activity decreases after storage if the enzyme particles are dissolved.

A suitable amount of the water-soluble salt is at least 1% w/w. The upper limit is the solubility limit of the salt, e.g., 10% w/w.

**Viscosity regulating agent and other ingredients**

In order to avoid sedimentation of the enzyme particles, the enzyme slurry of the invention comprises a viscosity regulating agent, such as a natural polymer. The viscosity regulating agent is selected from the group consisting of alginate, carrageenan, xanthan gum, guar gum, locust bean gum, welan gum, gellan gum, and microcrystalline cellulose.

The viscosity regulating agent is added to the enzyme slurry in an amount sufficient to avoid sedimentation of the enzyme particles after storage of the slurry. The desired time of storage (*e.g.*, four weeks) regulates how much of the viscosity regulating agent is needed. This can be determined by incubating samples with different levels of the viscosity regulating agent for a desired time, and then measuring the sedimentation profile, as shown in Example 2.

The viscosity regulating agent is added in an amount of at least 0.1% w/w, preferably at least 0.25% w/w, depending on the type of viscosity regulating agent. The upper limit is determined by the level of viscosity that can be tolerated in the application, *e.g.,* 2.5% w/w.

The enzyme slurry may also include preservation agents, such as phenoxy ethanol, sorbate, or benzoate, in order to avoid microbial contamination/degradation after storage.

Water is used as the carrier liquid in the enzyme slurry. The exact amount of water depends on the amounts of other components in the enzyme slurry, but the enzyme slurry comprises at least 50% w/w water. In an embodiment, the amount of water is at least 60% w/w, preferably at least 70% w/w, preferably at least 80% w/w, and most preferably at least 90%. In a preferred embodiment, the water content is 50-90% w/w, 60-90%, or 70-90%.

**Enzyme(s)**

The enzyme particles (see above) used in the enzyme slurry of the invention include one or more enzymes, in particular enzymes suitable for including in laundry or dishwash detergents (detergent enzymes), such as a protease (e.g., subtilisin or metalloprotease), lipase, cutinase, amylase, carbohydrase, cellulase, pectinase, mannanase, arabinase, galactanase, xanthanase, xylanase, DNAse, perhydrolase, oxidoreductase (e.g., laccase, peroxidase, peroxygenase and/or haloperoxidase).

Preferred detergent enzymes are protease (e.g., subtilisin or metalloprotease), lipase, amylase, lyase, cellulase, pectinase, mannanase, DNAse, perhydrolase, and oxidoreductases (e.g., laccase, peroxidase, peroxygenase and/or haloperoxidase); or combinations thereof.

More preferred detergent enzymes are protease (e.g., subtilisin or metalloprotease), lipase, amylase, cellulase, pectinase, and mannanase; or combinations thereof.

The enzyme particles may be included in the enzyme slurry by adding separate particles each containing one enzyme, or by adding a combined particle comprising two or more of these enzymes.

Proteases

The proteases for use in the present invention are serine proteases, such as subtilisins, metalloproteases and/or trypsin-like proteases. Preferably, the proteases are subtilisins or metalloproteases; more preferably, the proteases are subtilisins.

A serine protease is an enzyme which catalyzes the hydrolysis of peptide bonds, and in which there is an essential serine residue at the active site (White, Handler and Smith, 1973 "Principles of Biochemistry," Fifth Edition, McGraw-Hill Book Company, NY, pp. 271-272). Subtilisins include, preferably consist of, the I-S1 and I-S2 sub-groups as defined by Siezen et al., Protein Engng. 4 (1991) 719-737; and Siezen et al., Protein Science 6 (1997) 501-523. Because of the highly conserved structure of the active site of serine proteases, the subtilisin according to the invention may be functionally equivalent to the proposed sub-group designated subtilase by Siezen et al. (supra).

The subtilisin may be of animal, vegetable or microbial origin, including chemically or genetically modified mutants (protein engineered variants), preferably an alkaline microbial subtilisin. Examples of subtilisins are those derived from Bacillus, e.g., subtilisin Novo, subtilisin Carlsberg, subtilisin BPN', subtilisin 309, subtilisin 147 and subtilisin 168 (described in WO 89/06279) and Protease PD138 (WO 93/18140). Examples are described in WO 98/020115, WO 01/44452, WO 01/58275, WO 01/58276, WO 03/006602 and WO 04/099401.

Other examples of useful proteases are the variants described in WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 43, 57, 61, 62, 68, 76, 87, 95, 96, 97, 98, 99, 100, 101, 102, 103, 104, 106, 118, 120, 123, 128, 129, 130, 156, 158, 160, 161, 167, 170, 182, 185, 188, 191, 194, 195, 199, 204, 205, 206, 209, 212, 217, 218, 224, 232, 235, 236, 245, 248, 252, 261, 262, 274 and 275, using the BPN' numbering.

The protease may comprise a substitution at one or more positions corresponding to positions 171, 173, 175, 179, or 180 of SEQ ID NO: 3 of WO 2004/067737.

More preferred the protease variants may comprise one or more of the following substitutions: S3T, V4I, S9R, S9E, A15T, K27R, *36D, N43R, G61E, G61D, N62D, N62E, V68A, N76D, N87S,R, *97E, A98S, S99G,S99D, S99A, S99AD, S101E, S101D, S101G, S101M, S101N, S101R, S101H, S103A, V104I, V104Y, V104N, S106A, G118V, G118R, H120D, H120N, N123S, S128L, P129Q, S130A, S156D, A158E, G160D, G160P, S161E, Y167A, R170S, Q182E, N185E, S188E, Q191N, A194P, G195E, V199M, N204D, V205I, Y209W, S212G, L217Q, L217D, N218D, N218S, A232V, K235L, Q236H, Q245R, N252K, N261W, N261D, N261E, L262E, L262D T274A, R275H (using BPN' numbering).

Examples of commercially available proteases include those sold under the trade names Alcalase™, Relase™, Relase™ Ultra, Savinase™, Savinase™ Ultra, Duralase™, Durazym™, Everlase™, Primase™, Polarzyme™, Kannase™, Liquanase™, Liquanase™ Ultra, Ovozyme™, Coronase™, Coronase™ Ultra, Blaze™, Blaze Evity™ 100T, Blaze Evity™ 125T, Blaze Evity™ 150T, Neutrase™, Esperase™, Carnival™, Progress Uno™, and Progress Excel™ (Novozymes A/S); those sold under the tradename Maxatase™, Maxacal™, Puramax™, FN2™, FN3™, FN4™, Excellase™, Maxapem™, Purafect Ox™, Purafect OxP™, Effectenz™ P1050, Effectenz™ P1060, Excellenz™ P1000, Excellenz™ P1250, Eraser™, Preferenz™ P100, Purafect Prime™, Preferenz™ P110, Effectenz™ P1000, Purafect™, Effectenz™ P2000, Purafast™, Properase™, Opticlean™ and Optimase™ (Genencor/Danisco/DuPont); Axapem™ (Gist-Brocases N.V.); BLAP (sequence shown in Figure 29 of US5352604) and variants hereof (Henkel AG); and KAP (Bacillus alkalophilus subtilisin) from Kao.

Lyases

The lyase may be a pectate lyase derived from Bacillus, particularly B. licherniformis or B. agaradhaerens, or a variant derived of any of these, e.g. as described in US 6124127, WO 99/027083, WO 99/027084, WO 02/006442, WO 02/092741, WO 03/095638, Commercially available pectate lyases are XPect™; Pectawash™ and Pectaway™ (Novozymes A/S).

Mannanase

The mannanase may be an alkaline mannanase of Family 5 or 26. It may be a wild-type from Bacillus or Humicola, particularly B. agaradhaerens, B. licheniformis, B. halodurans, B. clausii, or H. insolens. Suitable mannanases are described in WO 99/064619. Commercially available mannanases are Mannaway™ (Novozymes A/S), and Mannastar™ (Dupont).

Cellulases

Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium, e.g., the fungal cellulases produced from Humicola insolens, Myceliophthora thermophila and Fusarium oxysporum disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, Carezyme™, Carezyme Premium™, Whitezyme™, and Celluclean™ (Novozymes A/S); Clazinase™, Revitalenz™, and Puradax HA™ (DuPont); Biotouch™ DCL and FCL (AB Enzymes); and KAC-500(B)™ (Kao Corporation).

Lipases and Cutinases

Suitable lipases and cutinases include those of bacterial or fungal origin. Chemically modified or protein engineered mutants are included. Examples include lipase from Thermomyces, e.g., from T. lanuginosus (previously named Humicola lanuginosa) as described in EP 258 068 and EP 305 216, cutinase from Humicola, e.g., H. insolens as described in WO 96/13580, a Pseudomonas lipase, e.g., from P. alcaligenes or P. pseudoalcaligenes (EP 218 272), P. cepacia (EP 331 376), P. stutzeri (GB 1,372,034), P. fluorescens, Pseudomonas sp. strain SD 705 (WO 95/06720 and WO 96/27002), P. wisconsinensis (WO 96/12012), a Bacillus lipase, e.g., from B. subtilis (Dartois et al., 1993, Biochemica et Biophysica Acta, 1131: 253-360), B. stearothermophilus (JP 64/744992) or B. pumilus (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079, WO 97/07202, WO 00/060063, WO 2007/087508 and WO 2009/109500.

Preferred commercially available lipase enzymes include Lipolase™, Lipolase Ultra™, and Lipex™; Lecitase™, Lipolex™; Lipoclean™, Lipoprime™ (Novozymes A/S). Other commercially available lipases include Lumafast (DuPont); Lipomax (Gist-Brocades/DuPont) and Bacillus sp. lipase from Solvay.

Amylases

Suitable amylases ($\alpha$ and/or $\beta$) include those of bacterial or fungal origin. Chemically modified or protein engineered mutants are included. Amylases include, for example, $\alpha$ amylases obtained from Bacillus, e.g., a special strain of Bacillus licheniformis, described in more detail in GB 1,296,839.

Examples of suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193. Other amylases which are suitable are hybrid alpha-amylase comprising residues 1-33 of the alpha-amylase derived from B. amyloliquefaciens shown in SEQ ID NO: 6 of WO 2006/066594 and residues 36-483 of the B. licheniformis alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, I201, A209 and Q264. Most preferred variants of the hybrid alpha-amylase comprising residues 1-33 of the alpha-amylase derived from B. amyloliquefaciens shown in SEQ ID NO: 6 of WO 2006/066594 and residues 36-483 of SEQ ID NO: 4 are those having the substitutions:

M 197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476. More preferred variants are those having a deletion in positions 181 and 182 or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Stainzyme™; Stainzyme Plus™; Duramyl™, Termamyl™, Termamyl Ultra™; Natalase™, Fungamyl™ and BAN™ (Novozymes A/S), Rapidase™ and Purastar™/Effectenz™, Powerase™, Amplify™, Amplify Prime™, Preferenz™ S100, and Preferenz™ S110 (DuPont).

Deoxyribonuclease (DNase)

Suitable deoxyribonucleases (DNases) are any enzyme that catalyzes the hydrolytic cleavage of phosphodiester linkages in the DNA backbone, thus degrading DNA. According to the invention, a DNase which is obtainable from a bacterium is preferred; in particular a DNase which is obtainable from a Bacillus is preferred; in particular a DNase which is obtainable from Bacillus subtilis or Bacillus licheniformis is preferred. Examples of such DNases are described in patent application WO 2011/098579 or in PCT/EP2013/075922.

Perhydrolases

Suitable perhydrolases are capable of catalyzing a perhydrolysis reaction that results in the production of a peracid from a carboxylic acid ester (acyl) substrate in the presence of a source of peroxygen (e.g., hydrogen peroxide). While many enzymes perform this reaction at low levels, perhydrolases exhibit a high perhydrolysis:hydrolysis ratio, often greater than 1. Suitable perhydrolases may be of plant, bacterial or fungal origin. Chemically modified or protein engineered mutants are included.

Examples of useful perhydrolases include naturally occurring Mycobacterium perhydrolase enzymes, or variants thereof. An exemplary enzyme is derived from Mycobacterium smegmatis. Such enzyme, its enzymatic properties, its structure, and variants thereof, are described in WO 2005/056782, WO 2008/063400, US 2008/145353, and US2007167344.

Oxidases/peroxidases

Suitable oxidases and peroxidases (or oxidoreductases) include various sugar oxidases, laccases, peroxidases and haloperoxidases.

Suitable peroxidases include those comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from Coprinopsis, e.g., from C. cinerea (EP 179,486), and variants thereof as those described in WO 93/24618, WO 95/10602, and WO 98/15257.

A peroxidase for use in the invention also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase is a chloroperoxidase. Preferably, the haloperoxidase is a vanadium haloperoxidase, i.e., a vanadate-containing haloperoxidase. In a preferred method of the present invention the vanadate-containing haloperoxidase is combined with a source of chloride ion.

Haloperoxidases have been isolated from many different fungi, in particular from the fungus group dematiaceous hyphomycetes, such as Caldariomyces, e.g., C. fumago, Alternaria, Curvularia, e.g., C. verruculosa and C. inaequalis, Drechslera, Ulocladium and Botrytis.

Haloperoxidases have also been isolated from bacteria such as Pseudomonas, e.g., P. pyrrocinia and Streptomyces, e.g., S. aureofaciens.

In an preferred embodiment, the haloperoxidase is derivable from Curvularia sp., in particular Curvularia verruculosa or Curvularia inaequalis, such as C. inaequalis CBS 102.42 as described in WO 95/27046; or C. verruculosa CBS 147.63 or C. verruculosa CBS 444.70 as described in WO 97/04102; or from Drechslera hartlebii as described in WO 01/79459, Dendryphiella salina as described in WO 01/79458, Phaeotrichoconis crotalarie as described in WO 01/79461, or Geniculosporium sp. as described in WO 01/79460.

An oxidase according to the invention include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be derived from plants, bacteria or fungi (including filamentous fungi and yeasts).

Suitable examples from fungi include a laccase derivable from a strain of Aspergillus, Neurospora, e.g., N. crassa, Podospora, Botrytis, Collybia, Fomes, Lentinus, Pleurotus, Trametes, e.g., T. villosa and T. versicolor, Rhizoctonia, e.g., R. solani, Coprinopsis, e.g., C. cinerea, C. comatus, C. friesii, and C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata (WO 92/01046), or Coriolus, e.g., C. hirsutus (JP 2238885).

Suitable examples from bacteria include a laccase derivable from a strain of Bacillus.

A laccase derived from Coprinopsis or Myceliophthora is preferred; in particular a laccase derived from Coprinopsis cinerea, as disclosed in WO 97/08325; or from Myceliophthora thermophila, as disclosed in WO 95/33836.

Examples of other oxidases include, but are not limited to, amino acid oxidase, glucose oxidase, lactate oxidase, galactose oxidase, polyol oxidase (e.g., WO2008/051491), and aldose oxidase. Oxidases and their corresponding substrates may be used as hydrogen peroxide generating enzyme systems, and thus a source of hydrogen peroxide. Several enzymes, such as peroxidases, haloperoxidases and perhydrolases, require a source of hydrogen peroxide. By studying EC 1.1.3._, EC 1.2.3._, EC 1.4.3._, and EC 1.5.3._ or similar classes (under the International Union of

Biochemistry), other examples of such combinations of oxidases and substrates are easily recognized by one skilled in the art.

Amino acid changes, as referenced above, may be of a minor nature, that is conservative amino acid substitutions or insertions that do not significantly affect the folding and/or activity of the protein; small deletions, typically of 1-30 amino acids; small amino or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for enzyme activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

The relatedness between two amino acid sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment).

**Enzyme stabilizers/inhibitors**

The enzyme(s), as described above, may be stabilized using conventional stabilizing agents, e.g., a polyol such as glycerol, (mono, di, or tri) propylene glycol, sugar alcohol, polypropylene glycol, and/or polyethylene glycol, preferably polyethylene glycol or polypropylene glycol with a molecular weight in the range of 200-1000; or compounds that act by temporarily reducing the activity of proteases (reversible inhibitors).

Thus, the composition of the invention may also include a protease inhibitor/stabilizer, which is a reversible inhibitor of protease activity, e.g., serine protease activity. Preferably, the protease inhibitor is a (reversible) subtilisin protease inhibitor. In particular, the protease inhibitor may be a peptide aldehyde, boric acid, or a boronic acid; or a derivative of any of these.

The protease inhibitor may have an inhibition constant to a serine protease, Ki (mol/L) of from 1E-12 to 1E-03; more preferred from 1E-11 to 1E-04; even more preferred from 1E-10 to 1E-05; even more preferred from 1E-10 to 1E-06; and most preferred from 1E-09 to 1E-07.

Boronic acids

The protease inhibitor may be boronic acid or a derivative thereof; preferably, phenylboronic acid or a derivative thereof. In an embodiment of the invention, the phenyl boronic acid derivative is of the following formula:

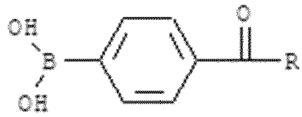

wherein R is selected from the group consisting of hydrogen, hydroxy, C1-C6 alkyl, substituted C1-C6 alkyl, C1-C6 alkenyl and substituted C1-C6 alkenyl. Preferably, R is hydrogen, CH3, CH3CH2 or CH3CH2CH2.

In a preferred embodiment, the protease inhibitor (phenyl boronic acid derivative) is 4-formyl-phenyl-boronic acid (4-FPBA).

In another particular embodiment, the protease inhibitor is selected from the group consisting of thiophene-2 boronic acid, thiophene-3 boronic acid, acetamidophenyl boronic acid, benzofuran-2 boronic acid, naphtalene-1 boronic acid, naphtalene-2 boronic acid, 2-FPBA, 3-FBPA, 4-FPBA, 1-thianthrene boronic acid, 4-dibenzofuran boronic acid, 5-methylthiophene-2 boronic, acid, thionaphtrene boronic acid, furan-2 boronic acid, furan-3 boronic acid, 4,4 biphenyl-diborinic acid, 6-hydroxy-2-naphtalene, 4-(methylthio) phenyl boronic acid, 4 (trimethylsilyl)phenyl boronic acid, 3-bromothiophene boronic acid, 4-methylthiophene boronic acid, 2-naphtyl boronic acid, 5-bromothiphene boronic acid, 5-chlorothiophene boronic acid, dimethylthiophene boronic acid, 2-bromophenyl boronic acid, 3-chlorophenyl boronic acid, 3-methoxy-2-thiophene, p-methyl-phenylethyl boronic acid, 2-thianthrene boronic acid, dibenzothiophene boronic acid, 4-carboxyphenyl boronic acid, 9-anthryl boronic acid, 3,5 dichlorophenyl boronic, acid, diphenyl boronic acidanhydride, o-chlorophenyl boronic acid, p-chlorophenyl boronic acid, m-bromophenyl boronic acid, p-bromophenyl boronic acid, p-flourophenyl boronic acid, p-tolyl boronic acid, o-tolyl boronic acid, octyl boronic acid, 1,3,5 trimethylphenyl boronic acid, 3-chloro-4-flourophenyl boronic acid, 3-aminophenyl boronic acid, 3,5-bis-(triflouromethyl) phenyl boronic acid, 2,4 dichlorophenyl boronic acid, and 4-methoxyphenyl boronic acid.

Further boronic acid derivatives suitable as protease inhibitors in the detergent composition are described in US 4,963,655, US 5,159,060, WO 95/12655, WO 95/29223, WO 92/19707, WO 94/04653, WO 94/04654, US 5442100, US 5488157 and US 5472628.

Peptide aldehyde or ketone

The protease stabilizer may have the formula: P-(A)y-L-(B)x-B0-R* wherein:

R* is H (hydrogen), $CH_3$, $CX_3$, $CHX_2$, or $CH_2X$. Preferably, R*=H so that the stabilizer is a peptide aldehyde with the formula P-(A)y-L-(B)x-B0-H;
X is a halogen atom, particularly F (fluorine);
B0 is a single amino acid residue with L- or D-configuration of the formula -NH-CH(R)-C(=O)-;
x is 1,2 or 3;
Bx is independently a single amino acid residue, each connected to the next B or to B0 via its C-terminal;
L is absent or independently a linker group of the formula -C(=O)-, -C(=O)-C(=O)-, -C(=S)-, -C(=S)-C(=S)- or -C(=S)-C(=O)-;
A is absent if L is absent or is independently a single amino acid residue connected to L via the N-terminal of the amino acid;
P is selected from the group consisting of hydrogen or if L is absent an N-terminal protection group;
y is 0, 1, or 2,
R is independently selected from the group consisting of $C_{1-6}$ alkyl, $C_{6-10}$ aryl or $C_{7-10}$ arylalkyl, optionally substituted with one or more, identical or different, substituent's R';
R' is independently selected from the group consisting of halogen, -OH, -OR", -SH, -SR", $-NH_2$, -NHR", $-NR"_2$, $-CO_2H$, $-CONH_2$, -CONHR", $-CONR"_2$, $-NHC(=N)NH_2$; and
R" is a $C_{1-6}$ alkyl group.
x may be 1, 2 or 3 and therefore B may be 1, 2 or 3 amino acid residues respectively. Thus, B may represent B1, B2-B1 or B3-B2-B1, where B3, B2 and B1 each represent one amino acid residue. y may be 0, 1 or 2 and therefore A may be absent, or 1 or 2 amino acid residues respectively having the formula A1 or A2-A1 wherein A2 and A1 each represent one amino acid residue.

B0 may be a single amino acid residue with L- or D-configuration, which is connected to H via the C-terminal of the

amino acid. B0 has the formula -NH-CH(R)-C(=O)-, wherein R is a $C_{1-6}$ alkyl, $C_{6-10}$ aryl or $C_{7-10}$ arylalkyl side chain, such as methyl, ethyl, propyl, isopropyl, butyl, isobutyl, phenyl or benzyl, and wherein R may be optionally substituted with one or more, identical or different, substituents R'. Particular examples of B0 are the D- or L-form of arginine (Arg), 3,4-dihydroxyphenylalanine, isoleucine (Ile), leucine (Leu), methionine (Met), norleucine (Nle), norvaline (Nva), phenylalanine (Phe), m-tyrosine, p-tyrosine (Tyr) and valine (Val). A particular embodiment is when B0 is leucine, methionine, phenylalanine, p-tyrosine and valine.

B1, which is connected to B0 via the C-terminal of the amino acid, may be an aliphatic, hydrophobic and/or neutral amino acid. Examples of B1 are alanine (Ala), cysteine (Cys), glycine (Gly), isoleucine (Ile), leucine (Leu), norleucine (Nle), norvaline (Nva), proline (Pro), serine (Ser), threonine (Thr) and valine (Val). Particular examples of B1 are alanine, glycine, isoleucine, leucine and valine. A particular embodiment is when B1 is alanine, glycine or valine.

If present, B2, which is connected to B1 via the C-terminal of the amino acid, may be an aliphatic, hydrophobic, neutral and/or polar amino acid. Examples of B2 are alanine (Ala), arginine (Arg), capreomycidine (Cpd), cysteine (Cys), glycine (Gly), isoleucine (Ile), leucine (Leu), norleucine (Nle), norvaline (Nva), phenylalanine (Phe), proline (Pro), serine (Ser), threonine (Thr), and valine (Val). Particular examples of B2 are alanine, arginine, capreomycidine, glycine, isoleucine, leucine, phenylalanine and valine. A particular embodiment is when B2 is arginine, glycine, leucine, phenylalanine or valine.

B3, which if present is connected to B2 via the C-terminal of the amino acid, may be a large, aliphatic, aromatic, hydrophobic and/or neutral amino acid. Examples of B3 are isoleucine (Ile), leucine (Leu), norleucine (Nle), norvaline (Nva), phenylalanine (Phe), phenylglycine, tyrosine (Tyr), tryptophan (Trp) and valine (Val). Particular examples of B3 are leucine, phenylalanine, tyrosine and tryptophan.

The linker group L may be absent or selected from the group consisting of -C(=O)-, - C(=O)-C(=O)-, -C(=S)-, -C(=S)-C(=S)- or -C(=S)-C(=O)-. Particular embodiments of the invention are when L is absent or L is a carbonyl group -C(=O)-.

A1, which if present is connected to L via the N-terminal of the amino acid, may be an aliphatic, aromatic, hydrophobic, neutral and/or polar amino acid. Examples of A1 are alanine (Ala), arginine (Arg), capreomycidine (Cpd), glycine (Gly), isoleucine (Ile), leucine (Leu), norleucine (Nle), norvaline (Nva), phenylalanine (Phe), threonine (Thr), tyrosine (Tyr), tryptophan (Trp) and valine (Val). Particular examples of A1 are alanine, arginine, glycine, leucine, phenylalanine, tyrosine, tryptophan and valine. A particular embodiment is when B2 is leucine, phenylalanine, tyrosine or tryptophan.

The A2 residue, which if present is connected to A1 via the N-terminal of the amino acid, may be a large, aliphatic, aromatic, hydrophobic and/or neutral amino acid. Examples of A2 are arginine (Arg), isoleucine (Ile), leucine (Leu), norleucine (Nle), norvaline (Nva), phenylalanine (Phe), phenylglycine, Tyrosine (Tyr), tryptophan (Trp) and valine (Val). Particular examples of A2 are phenylalanine and tyrosine.

The N-terminal protection group P (if present) may be selected from formyl, acetyl (Ac), benzoyl (Bz), trifluoroacetyl, methoxysuccinyl, aromatic and aliphatic urethane protecting groups such as fluorenylmethyloxycarbonyl (Fmoc), methoxycarbonyl (Moc), (fluoromethoxy)carbonyl, benzyloxycarbonyl (Cbz), t-butyloxycarbonyl (Boc) and adamantyloxycarbonyl; p-methoxybenzyl carbonyl, benzyl (Bn), p-methoxybenzyl (PMB), p-methoxyphenyl (PMP), methoxyacetyl, methylamino carbonyl, methylsulfonyl, ethylsulfonyl, benzylsulfonyl, methylphosphoramidyl (MeO-P(OH)(=O)) and benzylphosphoramidyl ($PhCH_2OP(OH)(=O)$).

In the case of a tripeptide aldehyde with a protection group (i.e. x=2, L is absent and A is absent), P is preferably acetyl, methoxycarbonyl, benzyloxycarbonyl, methylamino carbonyl, methylsulfonyl, benzylsulfonyl and benzylphosphoramidyl. In the case of a tetrapeptide aldehyde with a protection group (i.e. x=3, L is absent and A is absent), P is preferably acetyl, methoxycarbonyl, methylsulfonyl, ethylsulfonyl and methylphosphoramidyl.

Suitable peptide aldehydes are described in WO94/04651, WO95/25791, WO98/13458, WO98/13459, WO98/13460, WO98/13461, WO98/13462, WO07/141736, WO07/145963, WO09/118375, WO10/055052 and WO11/036153. More particularly, the peptide aldehyde may be Cbz-Arg-Ala-Tyr-H, Ac-Gly-Ala-Tyr-H, Cbz-Gly-Ala-Tyr-H, Cbz-Gly-Ala-Tyr-$CF_3$, Cbz-Gly-Ala-Leu-H, Cbz-Val-Ala-Leu-H, Cbz-Val-Ala-Leu-$CF_3$, Moc-Val-Ala-Leu-$CF_3$, Cbz-Gly-Ala-Phe-H, Cbz-Gly-Ala-Phe-$CF_3$, Cbz-Gly-Ala-Val-H, Cbz-Gly-Gly-Tyr-H, Cbz-Gly-Gly-Phe-H, Cbz-Arg-Val-Tyr-H, Cbz-Leu-Val-Tyr-H, Ac-Leu-Gly-Ala-Tyr-H, Ac-Phe-Gly-Ala-Tyr-H, Ac-Tyr-Gly-Ala-Tyr-H, Ac-Phe-Gly-Ala-Leu-H, Ac-Phe-Gly-Ala-Phe-H, Ac-Phe-Gly-Val-Tyr-H, Ac-Phe-Gly-Ala-Met-H, Ac-Trp-Leu-Val-Tyr-H, MeO-CO-Val-Ala-Leu-H, MeNCO-Val-Ala-Leu-H, MeO-CO-Phe-Gly-Ala-Leu-H, MeO-CO-Phe-Gly-Ala-Phe-H, $MeSO_2$-Phe-Gly-Ala-Leu-H, $MeSO_2$-Val-Ala-Leu-H, $PhCH_20$-P(OH)(O)-Val-Ala-Leu-H, $EtSO_2$-Phe-Gly-Ala-Leu-H, $PhCH_2SO_2$-Val-Ala-Leu-H, $PhCH_2O$-P(OH)(O)-Leu-Ala-Leu-H, $PhCH_2O$-P(OH)(O)-Phe-Ala-Leu-H, or MeO-P(OH)(O)-Leu-Gly-Ala-Leu-H. A preferred stabilizer for use in the liquid composition of the invention is Cbz-Gly-Ala-Tyr-H, or a hydrosulfite adduct thereof, wherein Cbz is benzyloxycarbonyl.

Further examples of such peptide aldehydes include α-MAPI, β-MAPI, Phe-C(=O)-Arg-Val-Tyr-H, Phe-C(=O)-Gly-Gly-Tyr-H, Phe-C(=O)-Gly-Ala-Phe-H, Phe-C(=O)-Gly-Ala-Tyr-H, Phe-C(=O)-Gly-Ala-L-H, Phe-C(=O)-Gly-Ala-Nva-H, Phe-C(=O)-Gly-Ala-Nle-H, Tyr-C(=O)-Arg-Val-Tyr-H, Tyr-C(=O)-Gly-Ala-Tyr-H, Phe-C(=S)-Arg-Val-Phe-H,

Phe-C(=S)-Arg-Val-Tyr-H, Phe-C(=S)-Gly-Ala-Tyr-H, Antipain, GE20372A, GE20372B, Chymostatin A, Chymostatin B, and Chymostatin C.

The protease stabilizer may be a hydrosulfite adduct of the peptide aldehyde described above, e.g. as described in WO 2013/004636. The adduct may have the formula P-(A)y-L-(B)x-N(H)-CHR-CH(OH)-$SO_3$M, wherein P, A, y, L, B, x and R are defined as above, and M is H or an alkali metal, preferably Na or K.

An aqueous solution of the hydrosulfite adduct may be prepared by reacting the corresponding peptide aldehyde with an aqueous solution of sodium bisulfite (sodium hydrogen sulfite, $NaHSO_3$); potassium bisulfite ($KHSO_3$) by known methods, e.g., as described in WO 98/47523; US 6,500,802; US 5,436,229; J. Am. Chem. Soc. (1978) 100, 1228; Org. Synth., Coll. vol. 7: 361.

Particularly preferred peptide aldehyde protease stabilizers have the formula P-B3-B2-B1-B0-H, or a hydrosulfite adduct having the formula P-B3-B2-B1-N(H)-CHR-CHOH-$SO_3$M, wherein

i) H is hydrogen;
ii) B0 is a single amino acid residue with L- or D-configuration of the formula -NH-CH(R)-C(=O)-;
iii) B1 and B2 are independently single amino acid residues;
iv) B3 is a single amino acid residue, or is absent;
v) R is independently selected from the group consisting of $C_{1-6}$ alkyl, $C_{6-10}$ aryl or $C_{7-10}$ arylalkyl optionally substituted with one or more, identical or different, substituents R';
vi) R' is independently selected from the group consisting of halogen, -OH, -OR", -SH, -SR", - $NH_2$, -NHR", -NR"$_2$, -$CO_2$H, -$CONH_2$, -CONHR", -CONR"$_2$, -NHC(=N)$NH_2$;
vii) R" is a $C_{1-6}$ alkyl group;
viii) P is an N-terminal protection group, preferably methoxycarbonyl (Moc) or benzyloxycarbonyl (Cbz); and
ix) M is H or an alkali metal, preferably Na or K.

In an even more preferred embodiment, the peptide aldehyde protease stabilizer has the formula P-B2-B1-B0-H or an adduct having the formula P-B2-B1-N(H)-CHR-CHOH-$SO_3$M, wherein

i) H is hydrogen;
ii) B0 is a single amino acid residue with L- or D-configuration of the formula -NH-CH(R)-C(=O)-;
iii)B1 and B2 are independently single amino acid residues;
iv) R is independently selected from the group consisting of $C_{1-6}$ alkyl, $C_{6-10}$ aryl or $C_{7-10}$ arylalkyl optionally substituted with one or more, identical or different, substituents R';
v) R' is independently selected from the group consisting of halogen, -OH, -OR", -SH, -SR", - $NH_2$, -NHR", -NR"$_2$, -$CO_2$H, -$CONH_2$, -CONHR", -CONR"$_2$, -NHC(=N)$NH_2$;
vi) R" is a $C_{1-6}$ alkyl group;
vii) P is an N-terminal protection group, preferably methoxycarbonyl (Moc) or benzyloxycarbonyl (Cbz); and
viii) M is H or an alkali metal, preferably Na or K.

Preferred embodiments of B0, B1, B2, B3, and P are as described above.

The molar ratio of the above-mentioned peptide aldehydes (or hydrosulfite adducts) to the protease may be at least 1:1 or 1.5:1, and it may be less than 1000:1, more preferred less than 500:1, even more preferred from 100:1 to 2:1 or from 20:1 to 2:1, or most preferred, the molar ratio is from 10:1 to 2:1.

Formate salts (e.g., sodium formate) and formic acid have also shown good effects as inhibitor of protease activity. Formate can be used synergistically with the above-mentioned protease inhibitors, as shown in WO 2013/004635. The formate salts may be present in the slurry composition in an amount of at least 0.1% w/w or 0.5% w/w, e.g., at least 1.0%, at least 1.2% or at least 1.5%. The amount is typically below 5% w/w, below 4% or below 3%.

In an embodiment, the protease is a metalloprotease and the inhibitor is a metalloprotease inhibitor, e.g., a protein hydrolysate based inhibitor (e.g., as described in WO 2008/134343).

## Detergent composition

The enzyme slurry of the invention may be added to, and thus form part of, any detergent composition in any form, such as liquid detergents, and soap and detergent bars (e.g., syndet bars). Soap and detergent bar compositions are described in more detail below.

The enzyme slurry, as described above, may be added to the liquid detergent composition in an amount corresponding to from 0.0001% to 5% (w/w) active enzyme protein (AEP); preferably from 0.0005% to 5%, more preferably from 0.0005% to 2%, more preferably from 0.0005% to 1%, even more preferably from 0.001% to 1%, and most preferably from 0.005% to 1% (w/w) active enzyme protein.

The liquid detergent composition has a physical form, which is not solid (or gas). It may be a pourable liquid, a paste, a pourable gel or a non-pourable gel. It may be either isotropic or structured, preferably isotropic. It may be a formulation useful for washing in automatic washing machines or for hand washing, or for (automatic) dish wash. It may also be a personal care product, such as a shampoo, toothpaste, or hand soap.

The liquid detergent composition typically contains at least 20% by weight of water, and up to 95% water, such as up to 70% water, up to 50% water, up to 40% water, or up to 30% water. Other types of liquids, including without limitation, alkanols, amines, diols, ethers and polyols may be included in an aqueous liquid detergent. An aqueous liquid detergent may contain from 0-30% organic solvent.

The choice of detergent components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

The choice of additional components is within the skill of the artisan and includes conventional ingredients, including the exemplary non-limiting components set forth below.

<u>Surfactants</u>

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and includes any conventional surfactant(s) known in the art. Any surfactant known in the art for use in detergents may be utilized.

When included therein the detergent will usually contain from about 1% to about 40% by weight, such as from about 5% to about 30%, including from about 5% to about 15%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein the detergent will usually contain from about 0.1% to about 10% by weight of a cationic surfactant. Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

When included therein the detergent will usually contain from about 0.2% to about 40% by weight of a non-ionic surfactant, for example from about 0.5% to about 30%, in particular from about 1% to about 20%, from about 3% to about 10%, such as from about 3% to about 5%, or from about 8% to about 12%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or *N*-acyl *N*-alkyl derivatives of glucosamine (glucamides, GA, or fatty acid glucamide, FAGA), as well as products available under the trade names SPAN and TWEEN, and combinations thereof.

When included therein the detergent will usually contain from about 0.1% to about 20% by weight of a semipolar surfactant. Non-limiting examples of semipolar surfactants include amine oxides (AO) such as alkyldimethylamineoxide, *N*-(coco alkyl)-*N,N*-dimethylamine oxide and N-(tallow-alkyl)-*N,N*-bis(2-hydroxyethyl)amine oxide, fatty acid alkanolamides and ethoxylated fatty acid alkanolamides, and combinations thereof.

When included therein the detergent will usually contain from about 0.1% to about 10% by weight of a zwitterionic surfactant. Non-limiting examples of zwitterionic surfactants include betaine, alkyldimethylbetaine, sulfobetaine, and combinations thereof.

Hydrotropes

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see for example review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-5% by weight, such as about 0.5 to about 5%, or about 3% to about 5%, of a hydrotrope. Any hydrotrope known in the art for use in detergents may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

Builders and Co-Builders

The detergent composition may contain about 0-65% by weight, such as about 5% to about 50% of a detergent builder or co-builder, or a mixture thereof. In a dish wash detergent, the level of builder is typically 40-65%, particularly 50-65%. The builder and/or co-builder may particularly be a chelating agent that forms water-soluble complexes with Ca and Mg ions. Any builder and/or co-builder known in the art for use in laundry detergents may be utilized. Non-limiting examples of builders include citrates, diphosphates (pyrophosphates), triphosphates such as sodium triphosphate (STP or STPP), carbonates such as sodium carbonate, ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI), and combinations thereof.

The detergent composition may also contain 0-50% by weight, such as about 5% to about 30%, of a detergent co-builder, or a mixture thereof. The detergent composition may include include a co-builder alone, or in combination with a builder, for example a citrate builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N,N'*-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), *N*-(2-hydroxyethyl)iminodiacetic acid (EDG), aspartic acid-*N*-monoacetic acid (ASMA), aspartic acid-*N,N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), α-alanine-*N, N*-diacetic acid (α-ALDA), serine-*N*, *N*-diacetic acid (SEDA), isoserine-*N*, *N*-diacetic acid (ISDA), phenylalanine-*N*, *N*-diacetic acid (PHDA), anthranilic acid-*N*, *N*-diacetic acid (ANDA), sulfanilic acid-*N*, *N*-diacetic acid (SLDA) , taurine-*N*, *N*-diacetic acid (TUDA) and sulfomethyl-*N*, *N*-diacetic acid (SMDA), *N*-(2-hydroxyethyl)-ethylidenediamine-*N*, *N'*, *N'*-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, *e.g.,* WO 09/102854, US 5977053.

Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide anti redeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine),

carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, *e.g.,* WO 2006/130575 and US 5,955,415. Salts of the above-mentioned polymers are also contemplated.

Fabric hueing agents

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO 2005/03274, WO 2005/03275, WO 2005/03276 and EP 1876226. The detergent composition preferably comprises from 0.0001 to 0.2% w/w fabric hueing agent. Suitable hueing agents are also disclosed in, *e.g.,* WO 2007/087257 and WO 2007/087243.

Adjunct materials

Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including the enzyme stabilizers/inhibitors mentioned above, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

Dispersants - The detergent compositions of the present invention can also contain dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

Dye Transfer Inhibiting Agents - The detergent compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

Fluorescent whitening agent - The detergent compositions of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl] benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2% w/w to upper levels of 0.5 or even 0.75% w/w.

Soil release polymers - The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore random graft co-polymers are suitable soil release polymers. Suitable graft co-polymers are described in more detail in WO 2007/138054, WO 2006/108856 and WO 2006/113314. Other soil release polymers are substituted polysaccharide structures especially substituted cellulosic structures such as modified cellulose deriviatives such as those described in EP 1867808 or WO 2003/040279. Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents - The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Rheology Modifiers are structurants or thickeners, as distinct from viscosity reducing agents. The rheology modifiers are selected from the group consisting of non-polymeric crystalline, hydroxy-functional materials, polymeric rheology modifiers which impart shear thinning characteristics to the aqueous liquid matrix of the composition. The rheology and viscosity of the detergent can be modified and adjusted by methods known in the art, for example as shown in EP 2169040.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

Bleaching Systems

Examples of liquid detergents combining bleach and enzymes include, e.g., US 5,275,753 and WO 99/00478. The detergent may contain 0-50% of a bleaching system. Any bleaching system known in the art for use in laundry detergents may be utilized. Suitable bleaching system components include bleaching catalysts such as MnTACN, photobleaches, bleach activators, sources of hydrogen peroxide such as sodium percarbonate and sodium perborates, preformed peracids and mixtures thereof.

**Soap and detergent bars**

Laundry soap and detergent bars may comprise surfactants (such as anionic synthetic surfactants), builders (such as complexing agents like EDTA and HEDP (1-hydroxyethane 1,1-diphosphonic acid)), polymeric soil release agents, detergent chelators, polyols (such as glycerol), pH controlling compounds, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as, but not limited to, mixers, millers, plodders, extruders, cutters and wrappers. The soap and detergent bar is not limited to preparation by any single method.

The enzyme particles may be present in the laundry soap bar in an amount in the range from 0.00001% to 2.0% w/w. Typical amounts are in the range from 0.0001% to 0.4%, preferably in the range 0.001% to 0.1%, most preferably in the range 0.004% to 0.08% by weight of the total composition of the laundry soap bar.

When the enzyme slurry is used in the manufacturing of a soap or detergent bar, it is an advantage not to dissolve the enzyme particles (*e.g.,* enzyme crystals) in the manufacturing process. Soap and detergent bars with such undissolved enzyme particles exhibits a higher residual enzyme activity after storage, as compared to soap and detergent bars made using dissolved enzyme.

Polyols

Polyols useful as ingredients in laundry soap bars are characterized by solubility in water, carbon backbones of length between C2 and C6 and multiple hydroxyl groups, preferably containing between 2 and 6 hydroxyl groups per molecule.

Polyols useful herein include, but are not limited to 1,2-butanediol, 3-chloro-1,2-propanediol, ethylenediol, 1,2-hexanediol, glycerol (glycerine), mannose, propylene glycol, sorbitol, sucrose and mixtures thereof. The polyol level will usually be between 0.5% and 10%, and preferably between 1% and 5%, and more preferably between 1.5% and 3% by weight of the final bar composition.

Glycerine

During soap manufacturing glycerine is either being removed due to commercial reasons or it is left in the soap. Therefore soaps can be described as either high glycerine or low glycerine soaps. Low glycerine soaps comprises from 0 to 2% glycerine, and if more glycerine is needed it may be added in the manufacturing of the soap while the soap is still liquid to ensure proper uptake on or to soap noodles. In a particular embodiment, the laundry soap bar comprises from 1 to 6% glycerine. In a more particular embodiment of the present invention the laundry soap bar comprises from 2% to 5% glycerine by weight of total laundry soap bar. In a most preferred embodiment the laundry soap bar comprises about 4% glycerine.

pH controlling compound

It has been found that it may be necessary to lower the pH to improve stability of the enzyme. The pH controlling agent may be any suitable compound which is capable of controlling the pH. In a particular embodiment, the pH controlling agent is an acid.

In a particular embodiment the pH controlling compound may be selected from the group consisting of a fatty acid, acetic acid, aconitic acid, adipic acid, arachidic acid, arachidonic acid, aspartic acid, behenic acid, butyric acid, capric acid, capronic acid, cerotic acid, citric acid, formic acid, fumaric acid, glutamic acid, glutaric acid, glyceric acid, glyceric acid-3-phosphate, glyoxylic acid, isocitric acid, $\alpha$-ketoglutaric acid, lactic acid, lauric aicd, linoleic acid, linolenic acid, maleic acid, malic acid, malonic acid, myristic acid, oleic acid, oxalic acid, oxaloacetic acid, palmitic acid, palmitoleic acid, phosphatidic acid, phosphoenolpyruvic acid, pimelic acid, propionic acid, pyruvic acid, stearic acid, succinic acid, tartraic acid and valeric acid.

The pH controlling agent will usually be present at a level between 0.01% and 8%, preferably between 0.1% and 6%, more preferably between 0.2% and 2% by weight of the final bar composition.

Moisture content

Moisture provides the laundry soap bar with acceptable feel and other physical characteristics. Moisture can be added to the premix by being included with an ingredient and/or as free water added to the premix. The laundry soap bar contains less than 30% w/w water (moisture) content of the final laundry soap bar, or from 10% to 30%, preferably from 20% to 30% water (moisture) content of the final laundry soap bar.

Soap

Soap includes water soluble salts of higher fatty acids. The soap may be from an animal and/or vegetable source. Soap can be made by direct saponification of fats and oils or by neutralisation of free fatty acids. Suitable soaps are sodium, potassium, ammonium, and alkyloammonium salts of higher fatty acids containing from about 8 to about 24 carbon atoms, such as from 12 to about 18 carbon atoms. In a particular embodiment, the soap is selected from sodium and potassium salts of mixtures of fatty acid derived from coconut oil and tallow, such as sodium or potassium tallow and palm oil. Furthermore, nut oil, such as coconut or palm kernel oil, may be added in amounts of 5-30% by weight of the final bar.

Synthetic surfactant

Synthetic anionic surfactants, which are suitable for use in laundry soap bars, include the water-soluble salts, preferably the alkali metal, ammonium and alkyl ammonium salts of organic sulphuric reaction products having in their molecular structure an alkyl group containing from about 10 to about 20 carbon atoms and a sulfonic acid or sulphuric acid ester group. Examples of this group of synthetic surfactants are the sodium and potassium alkyl sulfates, especially those obtained by sulfating the higher alcohols (C8-18 carbon atoms) such as those produced by reducing the glycerides of

tallow or coconut oil; and the sodium and potassium alkyl benzene sulfonates in which the alkyl group contains from about 9 to about 15 carbon atoms, in straight chain or branched chain configuration. Especially valuable are the linear straight chain alkyl benzene sulfonates (LAS) in which the average number of carbon atoms in the alkyl group is from about 11-13, abbreviated as C11-13 LAS. The alkali metal salts, particularly the sodium salts of these surfactants are preferred.

Other examples of an anionic synthetic detergent suitable for use herein are the sodium alkyl glyceryl ether sulfonates (AES), especially those ethers of higher alcohols derived from tallow and coconut oil; sodium coconut oil fatty acid monoglyceride sulfonates and sulfates; and sodium or potassium salts of alkyl ethylene oxide ether sulfates containing about 1 to about 10 units ethylene oxide per molecule and wherein the alkyl group contains from about 10 to about 20 carbon atoms.

In addition, a suitable anionic synthetic detergent also includes the water soluble salts of ester of alpha-sulphonated fatty acids containing from about 6 to about 20 carbon atoms in the fatty acid group and from about 1 to about 10 carbon atoms in the ester group; water soluble salts of 2-acyloxyalkane-1-sulfonic acids containing from about 2 to about 9 carbon atoms in the acyl group and from 9 to about 23 carbon atoms in the alkane moiety; water soluble salts of olefin and paraffin sulfonates containing from about 12 to about 20 carbon atoms; and beta-alkyloxy alkane sulfonates containing from about 1 to about 3 carbon atoms in the alkyl group and from about 8 to about 20 carbon atoms in the alkane moiety.

Preferred anionic synthetic surfactant examples are C10-18 alkyl sulfates (AS), C10-18 linear alkyl benzene sulfonates (LAS), C10-14 alkyl glyceryl ether sulfonates (AES), and mixtures thereof.

An example of ingredients comprised in a conventional laundry soap bar (syndet bar) is: linear alkyl benzene sulfonate, coco fatty alcohol sulphate, soda ash, calcium carbonate, coco fatty alcohol, TiO2, cellulase, diethylenetriamine (methylenephosphonic acid), coco monoethanolamide, fluorecent whitening agents, substituted methylcellulose, perfume, moisture.

An example of detergent ingredients comprised in a conventional laundry soap bar (combo bar) is:
LAS, soap, cellulase, protease, borax, sodium silicate, citric acid, sodium carbonate, glycerol, propylene glycerol, sugar (sorbitol), $MgSO_4$, soda ash, talc, moisture.

## EXAMPLES

Protease inhibitor (also simply referred to as 'inhibitor' in the example): Cbz-Gly-Ala-NHCH($CH_2C_6H_4$pOH) CH(OH)($SO_3$)Na, wherein Cbz is benzyloxycarbonyl (hydrosulfite/bisulfite form of a peptide aldehyde).
Protease1 as used in the following Examples is Savinase™, which has the amino acid sequence as shown in SEQ ID NO: 1. Protease2 is another protease, which has the amino acid sequence as shown in SEQ ID NO: 2. 'Active enzyme protein' means enzyme protein which exhibits catalytic activity.
The procedure of enzyme slurry preparation can be divided into three steps:

1. Mix all ingredients except the viscosity regulating agent and enzyme particles in water to make a premixture. Stir at room temperature with anchor stirrer.
2. Add the viscosity regulating agent gradually over 10 min, while stirring with gradually increasing speed up to 800 rpm. Keep stirring at room temperature for 1 h with anchor stirrer.
3. Add the enzyme particles concentrate to the mixture. After stirring this final mixture at room temperature for 16 h at 800 rpm with anchor stirrer, the product is ready.

## EXAMPLE 1

### Effects of xanthan gum concentrations on crystal enzyme slurry sedimentation profile

Crystal protease slurry mixtures were prepared with various concentrations of xanthan gum. The formulation of each mixture is indicated in Table 1.

Table 1. Formulation of Mixture with different xanthan gum concentrations

| | Mixture 1 | Mixture 2 | Mixture 3 | Mixture 4 | Mixture 5 |
|---|---|---|---|---|---|
| Protease1 active enzyme protein | 10% | 10% | 10% | 10% | 10% |
| Xanthan gum | 0% | 0.063% | 0.125% | 0.25% | 0.50% |
| Potassium acetate | 2.5% | 2.5% | 2.5% | 2.5% | 2.5% |
| Protease inhibitor | 0.43% | 0.43% | 0.43% | 0.43% | 0.43% |

(continued)

| | Mixture 1 | Mixture 2 | Mixture 3 | Mixture 4 | Mixture 5 |
|---|---|---|---|---|---|
| Phenoxy ethanol | 0.5% | 0.5% | 0.5% | 0.5% | 0.5% |

After storage at 25°C for 8 weeks, the sedimentation of protease particles was observed, and evaluated by calculating the percentage of clear liquid (supernatant length against full slurry length) in the test vial (Table 2). The results showed that a low sedimentation can be obtained using a xanthan gum concentration of about 0.125% w/w. Almost no sedimentation occurs when higher amounts of xanthan gum is used. Mixture 1 and mixture 2 are not in accordance with the invention.

Table 2. Sedimentation profile of crystal savinase slurry with various concentrations of xanthan gum (25°C)

| % Clear liquid | Mixture 1 | Mixture 2 | Mixture 3 | Mixture 4 | Mixture 5 |
|---|---|---|---|---|---|
| 1 week | 41% | 12% | 0% | 0% | 0% |
| 2 weeks | 62% | 27% | 10% | 0% | 0% |
| 4 weeks | 66% | 38% | 7% | 3% | 0% |
| 8 weeks | 67% | 33% | 12% | 2% | 0% |

**EXAMPLE 2**

**Fast dissolution of enzyme slurry in liquid detergents with additives**

In order to reduce processing time in detergent manufacturing, it is desirable to achieve a fast dissolution of the enzyme slurry in commercial liquid detergents. Crystal protease slurry with protease inhibitor and/or PEG400 as additives were investigated to evaluate the time required to achieve complete dissolution in commercial detergents (Table 3).

Table 3. Formulation of crystal protease slurry with or without additives.

| | Mixture 5 | Mixture 6 | Mixture 7 | Mixture 8 |
|---|---|---|---|---|
| Protease1 active enzyme protein | 10% | 10% | 10% | 10% |
| Xanthan gum | 0.50% | 0.50% | 0.50% | 0.50% |
| Potassium acetate | 2.5% | 2.5% | 2.5% | 2.5% |
| Protease inhibitor | 0.43% | 0% | 0.43% | 0% |
| Phenoxy ethanol | 0.5% | 0.5% | 0.5% | 0.5% |
| PEG400 | 0% | 0% | 5% | 5% |

The results showed addition of DSAA could dramatically reduce dissolution time of crystal savinase slurry in three liquid detergents, in which two of them were commercially available on the Chinese market (Table 4). Increase of temperature to 50°C could reduce dissolution time further within 1h in all given liquid detergents. It is also noted crystal enzyme slurry could dissolve completely in the given detergents after days without stirring (data not shown here). Addition of PEG has no improving effect in terms of dissolution speed.

Table 4. Dissolution time of crystal protease slurry in liquid detergents (with stirring)

| | Temp. | Mixture 5 | Mixture 6 | Mixture 7 | Mixture 8 |
|---|---|---|---|---|---|
| Detergent 1 | 25°C | <1h | >12h | N.A. | N.A. |
| | 50°C | <1h | N.A. | N.A. | N.A. |
| Detergent 2 | 25°C | <2h | >12h | <3h | >12h |
| | 50°C | <0.5h | N.A. | N.A. | N.A. |

(continued)

| | Temp. | Mixture 5 | Mixture 6 | Mixture 7 | Mixture 8 |
|---|---|---|---|---|---|
| Detergent 3 | 25°C | <2h | >12h | <3h | >12h |
| | 50°C | <1h | N.A. | N.A. | N.A. |

N A · Not Available
Detergent 1 is CN20, standard liquid detergent base of China, based on standard recipe.
Detergent 2 is Liby Quanxiao detergent, commercial liquid detergent bought in Supermarket in January of 2015.
Detergent 3 is Blue Moon Deep Clean detergent, commercial liquid detergent bought in Supermarket in January of 2015.

**EXAMPLE 3**

**Salt effects on sedimentation profile of crystal enzyme slurry**

Enzyme slurry requires water-soluble salts, such as potassium or sodium salts, to prevent dissolution of the enzyme particles after storage in the slurry. Different types and concentrations of salts were investigated to elucidate their effects on sedimentation profile of crystal protease slurry (Table 5).

Table 5. Formulation of Mixtures of crystal Savinase slurry with salts.

| | Mixture 5 | Mixture 9 | Mixture 10 | Mixture 11 |
|---|---|---|---|---|
| Protease1 crystals (active enzyme protein) | 10% | 10% | 10% | 10% |
| Xanthan gum | 0.50% | 0.50% | 0.50% | 0.50% |
| Potassium acetate | 2.5% | 5% | 10% | 0% |
| Sodium formate | 0% | 0% | 0% | 5% |
| Protease inhibitor | 0.43% | 0% | 0.43% | 0% |
| Phenoxy ethanol | 0.5% | 0.5% | 0.5% | 0.5% |

The results showed that 2.5%, 5%, 10% potassium acetate and 5% sodium formate had no differences on their sedimentation profiles. Sodium formate showed an equivalent effect on sedimentation profile as potassium acetate at the same concentration (based on visual inspection).

**EXAMPLE 4**

**Per se stability of enzyme slurry**

The *per se* stability of crystal protease slurry in various formulations was evaluated. This showed they were all very stable, and equivalent to commercial liquid protease products (Table 6). Savinase Evity 16L contains a protease inhibitor and Savinase 16L contains no inhibitor.

Table 6. Per se stability of crystal protease slurry in various formulations.

| Storage conditions | Residual protease activity (%) | | | | | |
|---|---|---|---|---|---|---|
| | Savinase 16 L | Savinase Evity 16L | Mixture 1 | Mixture 5 | Mixture 7 | Mixture 11 |
| 4w 25°C | 100% | 100% | 102% | 96% | 97% | 108% |
| 4w 40°C | 99% | 99% | 102% | 97% | 101% | 104% |
| 4w 50°C | 95% | 99% | 104% | N.A. | 100% | N.A. |
| 13w 25°C | 100% | 98% | 96% | 100% | 105% | 97% |
| 13w 40°C | 98% | 98% | 95% | 100% | 107% | 100% |
| 13w 50°C | 84% | 98% | N.A. | 98% | 105% | 98% |

(continued)

| Storage conditions | Residual protease activity (%) | | | | | |
|---|---|---|---|---|---|---|
| | Savinase 16 L | Savinase Evity 16L | Mixture 1 | Mixture 5 | Mixture 7 | Mixture 11 |
| 26w 25°C | 100% | 97% | 99% | N.A. | N.A. | N.A. |
| 26w 40°C | 97% | 96% | 93% | N.A. | N.A. | N.A. |
| N.A.: Not Available. | | | | | | |

**Claims**

**1.** An enzyme slurry composition comprising

(a) enzyme particles;
(b) a water-soluble salt selected from the group consisting of sodium sulfate, potassium sulfate, magnesium sulfate, zinc sulfate, ammonium sulfate, sodium nitrate, potassium nitrate, magnesium nitrate, zinc nitrate, ammonium nitrate, sodium chloride, potassium chloride, magnesium chloride, zinc chloride, ammonium chloride, sodium acetate, potassium acetate, magnesium acetate, zinc acetate, ammonium acetate, sodium citrate, potassium citrate, magnesium citrate, zinc citrate, ammonium citrate, sodium formate, potassium formate, magnesium formate, zinc formate, and ammonium formate;
(c) at least 0.1% w/w of xanthan gum, guar gum, locust bean gum, welan gum, gellan gum, alginate, carrageenan, or microcrystalline cellulose; and
(d) at least 50% w/w of water;

wherein the enzyme particles consist of enzyme crystals or a spray dried enzyme composition, and wherein the enzyme slurry comprises at least 1% w/w of active enzyme protein.

**2.** The enzyme slurry of claim 1, which comprises the xanthan gum, guar gum, locust bean gum, welan gum, gellan gum, alginate, carrageenan, or microcrystalline cellulose in an amount of 0.25-2.5% w/w.

**3.** The enzyme slurry of any of the preceding claims, which comprises the water-soluble salt in an amount of 1-10% w/w.

**4.** The enzyme slurry of any of the preceding claims, which comprises the enzyme particles in an amount of at least 5% w/w.

**5.** The enzyme slurry of any of the preceding claims, which comprises at least 5% w/w active enzyme protein.

**6.** The enzyme slurry of any of the preceding claims, wherein the enzyme is selected from a protease (*e.g.,* subtilisin or metalloprotease), lipase, cutinase, amylase, carbohydrase, cellulase, pectinase, mannanase, arabinase, galactanase, xanthanase, xylanase, DNase, perhydrolase, oxidoreductase (*e.g.,* laccase, peroxidase, peroxygenase and/or haloperoxidase); preferably a protease, lipase, amylase, cellulase, pectinase, mannanase, and/or DNase.

**7.** The enzyme slurry of any of the preceding claims, which further comprises a polyol selected from the group consisting of glycerol, (mono, di, or tri) propylene glycol, sugar alcohol, polypropylene glycol, and/or polyethylene glycol, preferably polyethylene glycol or polypropylene glycol with a molecular weight in the range of 200-800.

**8.** The enzyme slurry of any of the preceding claims, wherein the water content is 50-90% w/w.

**9.** The enzyme slurry of any of the preceding claims, which further comprises an enzyme inhibitor.

**10.** The enzyme slurry of claim 9, wherein the enzyme is a protease and the inhibitor is a peptide aldehyde or a derivative thereof, or a boronic acid.

**11.** The enzyme slurry of claim 9 or 10, wherein the protease inhibitor is Cbz-Arg-Ala-Tyr-H, Ac-Gly-Ala-Tyr-H, Cbz-Gly-Ala-Tyr-H, Cbz-Gly-Ala-Tyr-CF3, Cbz-Gly-Ala-Leu-H, Cbz-Val-Ala-Leu-H, Cbz-Val-Ala-Leu-CF3, Moc-Val-Ala-Leu-$CF_3$, Cbz-Gly-Ala-Phe-H, Cbz-Gly-Ala-Phe-CF3, Cbz-Gly-Ala-Val-H, Cbz-Gly-Gly-Tyr-H, Cbz-Gly-Gly-Phe-H, Cbz-Arg-Val-Tyr-H, Cbz-Leu-Val-Tyr-H, Ac-Leu-Gly-Ala-Tyr-H, Ac-Phe-Gly-Ala-Tyr-H, Ac-Tyr-Gly-Ala-Tyr-H, Ac-Phe-Gly-Ala-Leu-H, Ac-Phe-Gly-Ala-Phe-H, Ac-Phe-Gly-Val-Tyr-H, Ac-Phe-Gly-Ala-Met-H, Ac-Trp-Leu-Val-Tyr-H, MeO-CO-Val-Ala-Leu-H, MeNCO-Val-Ala-Leu-H, MeO-CO-Phe-Gly-Ala-Leu-H, MeO-CO-Phe-Gly-Ala-Phe-H, MeSO2-Phe-Gly-Ala-Leu-H, MeSO2-Val-Ala-Leu-H, PhCH2OP(OH)(O)-Val-Ala-Leu-H, EtSO2-Phe-Gly-Ala-Leu-H, PhCH2SO2-Val-Ala-Leu-H, PhCH2OP(OH)(O)-Leu-Ala-Leu-H, PhCH2O-P(OH)(O)-Phe-Ala-Leu-H, or MeO-P(OH)(O)-Leu-Gly-Ala-Leu-H or a hydrosulfite adduct of any of these, wherein Cbz is benzyloxycarbonyl and Moc is methoxycarbonyl; preferably wherein the protease inhibitor is Cbz-Gly-Ala-Tyr-H or Moc-Val-Ala-Leu-H, or a hydrosulfite adduct thereof, wherein Cbz is benzyloxycarbonyl and Moc is methoxycarbonyl.

**12.** A method for preparing an enzyme-containing detergent composition, comprising adding an enzyme slurry according to any of the preceding claims to a composition comprising one or more detergent ingredients, such as surfactants and/or builders.

**13.** The method of claim 12, wherein the detergent is a soap or detergent bar.

**Patentansprüche**

**1.** Enzymaufschlämmungszusammensetzung, umfassend

(a) Enzympartikel;
(b) ein wasserlösliches Salz, ausgewählt aus der Gruppe bestehend aus Natriumsulfat, Kaliumsulfat, Magnesiumsulfat, Zinksulfat, Ammoniumsulfat, Natriumnitrat, Kaliumnitrat, Magnesiumnitrat, Zinknitrat, Ammoniumnitrat, Natriumchlorid, Kaliumchlorid, Magnesiumchlorid, Zinkchlorid, Ammoniumchlorid, Natriumacetat, Kaliumacetat, Magnesiumacetat, Zinkacetat, Ammoniumacetat, Natriumcitrat, Kaliumcitrat, Magnesiumcitrat, Zinkcitrat, Ammoniumcitrat, Natriumformiat, Kaliumformiat, Magnesiumformiat, Zinkformiat und Ammoniumformiat;
(c) mindestens 0,1% Gew./Gew. Xanthangummi, Guargummi, Carubin, Welangummi, Gellangummi, Alginat, Carrageen oder mikrokristalline Cellulose; und
(d) mindestens 50% Gew./Gew. Wasser;

wobei die Enzympartikel aus Enzymkristallen oder einer sprühgetrockneten Enzymzusammensetzung bestehen, und wobei die Enzymaufschlämmung mindestens 1% Gew./Gew. aktives Enzymprotein umfasst.

**2.** Enzymaufschlämmung nach Anspruch 1, die Xanthangummi, Guargummi, Carubin, Welangummi, Gellangummi, Alginat, Carrageen oder mikrokristalline Cellulose in einer Menge von 0,25-2,5% Gew./Gew. umfasst.

**3.** Enzymaufschlämmung nach einem beliebigen der voranstehenden Ansprüche, die das wasserlösliche Salz in einer Menge von 1-10% Gew./Gew. umfasst.

**4.** Enzymaufschlämmung nach einem beliebigen der voranstehenden Ansprüche, die die Enzympartikel in einer Menge von mindestens 5% Gew./Gew. umfasst.

**5.** Enzymaufschlämmung nach einem beliebigen der voranstehenden Ansprüche, die mindestens 5% Gew./Gew. aktives Enzymprotein umfasst.

**6.** Enzymaufschlämmung nach einem beliebigen der voranstehenden Ansprüche, wobei das Enzym aus einer Protease (z. B. Subtilisin oder Metalloprotease), Lipase, Cutinase, Amylase, Carbohydrase, Cellulase, Pectinase, Mannanase, Arabinase, Galactanase, Xanthanase, Xylanase, DNase, Perhydrolase, Oxidoreduktase (z. B. Laccase, Peroxidase, Peroxygenase und/oder Haloperoxidase) ausgewählt ist; vorzugsweise einer Protease, Lipase, Amylase, Cellulase, Pektinase, Mannanase und/oder DNase.

**7.** Enzymaufschlämmung nach einem beliebigen der voranstehenden Ansprüche, die des Weiteren ein Polyol umfasst, das aus der Gruppe bestehend aus Glycerin, (Mono-, Di- oder Tri-)Propylenglykol, Zuckeralkohol, Polypropylenglykol und/oder Polyethylenglykol ausgewählt ist, vorzugsweise Polyethylenglykol oder Polypropylenglykol mit einem Molekulargewicht im Bereich von 200-800.

**8.** Enzymaufschlämmung nach einem beliebigen der voranstehenden Ansprüche, wobei der Wassergehalt 50-90% Gew./Gew. ist.

**9.** Enzymaufschlämmung nach einem beliebigen der voranstehenden Ansprüche, die des Weiteren einen Enzyminhibitor umfasst.

**10.** Enzymaufschlämmung nach Anspruch 9, wobei das Enzym eine Protease ist, und der Inhibitor ein Peptidaldehyd oder ein Derivat davon oder eine Borsäure ist.

**11.** Enzymaufschlämmung nach Anspruch 9 oder 10, wobei der Proteaseinhibitor Cbz-Arg-Ala-Tyr-H, Ac-Gly-Ala-Tyr-H, Cbz-Gly-Ala-Tyr-H, Cbz-Gly-Ala-Tyr-CF3, Cbz-Gly-Ala-Leu-H, Cbz-Val-Ala-Leu-H, Cbz-Val-Ala-Leu-CF3, Moc-Val-Ala-Leu-$CF_3$, Cbz-Gly-Ala-Phe-H, Cbz-Gly-Ala-Phe-CF3, Cbz-Gly-Ala-Val-H, Cbz-Gly-Gly-Tyr-H, Cbz-Gly-Gly-Phe-H, Cbz-Arg-Val-Tyr-H, Cbz-Leu-Val-Tyr-H, Ac-Leu-Gly-Ala-Tyr-H, Ac-Phe-Gly-Ala-Tyr-H, Ac-Tyr-Gly-Ala-Tyr-H, Ac-Phe-Gly-Ala-Leu-H, Ac-Phe-Gly-Ala-Phe-H, Ac-Phe-Gly-Val-Tyr-H, Ac-Phe-Gly-Ala-Met-H, Ac-Trp-Leu-Val-Tyr-H, MeO-CO-Val-Ala-Leu-H, MeNCO-Val-Ala-Leu-H, MeO-CO-Phe-Gly-Ala-Leu-H, MeO-CO-Phe-Gly-Ala-Phe-H, MeSO2-Phe-Gly-Ala-Leu-H, MeSO2-Val-Ala-Leu-H, PhCH2O-P(OH)(O)-Val-Ala-Leu-H, EtSO2-Phe-Gly-Ala-Leu-H, PHCH2SO2-Val-Ala-Leu-H, PhCH2O-P(OH)(O)-Leu-Ala-Leu-H, PhCH2O-P(OH)(O)-Phe-Ala-Leu-H oder MeO-P(OH)(O)-Leu-Gly-Ala-Leu-H oder ein Hydrosulfitaddukt beliebiger davon ist, wobei Cbz Benzyloxycarbonyl und Moc Methoxycarbonyl ist; vorzugsweise wobei der Proteaseinhibitor Cbz-Gly-Ala-Tyr-H oder Moc-Val-Ala-Leu-H oder ein Hydrosulfitaddukt davon ist, wobei Cbz Benzyloxycarbonyl und Moc Methoxycarbonyl ist.

**12.** Verfahren zum Herstellen einer enzymhaltigen Detergenszusammensetzung, umfassend Hinzufügen einer Enzymaufschlämmung gemäß einem beliebigen der voranstehenden Ansprüche zu einer Zusammensetzung, die einen oder mehrere Detergensbestandteile, wie oberflächenaktive Mittel und/oder Gerüststoffe, umfasst.

**13.** Verfahren nach Anspruch 12, wobei das Detergens eine Seife oder ein Detergensriegel ist.

## Revendications

**1.** Composition de suspension enzymatique comprenant :

(a) des particules enzymatiques ;
(b) un sel hydrosoluble choisi dans le groupe consistant en sulfate de sodium, sulfate de potassium, sulfate de magnésium, sulfate de zinc, sulfate d'ammonium, nitrate de sodium, nitrate de potassium, nitrate de magnésium, nitrate de zinc, nitrate d'ammonium, chlorure de sodium, chlorure de potassium, chlorure de magnésium, chlorure de zinc, chlorure d'ammonium, acétate de sodium, acétate de potassium, acétate de magnésium, acétate de zinc, acétate d'ammonium, citrate de sodium, citrate de potassium, citrate de magnésium, citrate de zinc, citrate d'ammonium, formiate de sodium, formiate de potassium, formiate de magnésium, formiate de zinc, et formiate d'ammonium ;
(c) au moins 0,1 % p/p de gomme xanthane, gomme guar, gomme de graines de caroube, gomme Welan, gomme gellane, alginate, carraghénane, ou cellulose microcristalline ; et
(d) au moins 50 % p/p d'eau ;

dans laquelle les particules enzymatiques se composent de cristaux d'enzyme ou d'une composition enzymatique séchée par pulvérisation, et dans laquelle la suspension enzymatique comprend au moins 1 % p/p de protéine enzymatique active.

**2.** Suspension enzymatique selon la revendication 1, qui comprend de la gomme xanthane, gomme guar, gomme de graines de caroube, gomme Welan, gomme gellane, alginate, carraghénane, ou cellulose microcristalline en une quantité de 0,25 à 2,5 % p/p.

**3.** Suspension enzymatique selon l'une quelconque des revendications précédentes, qui comprend le sel hydrosoluble en une quantité de 1 à 10 % p/p.

**4.** Suspension enzymatique selon l'une quelconque des revendications précédentes, qui comprend les particules enzymatiques en une quantité d'au moins 5 % p/p.

**5.** Suspension enzymatique selon l'une quelconque des revendications précédentes, qui comprend au moins 5 % p/p de protéine enzymatique active.

6. Suspension enzymatique selon l'une quelconque des revendications précédentes, dans laquelle l'enzyme est choisie parmi une protéase (par exemple subtilisine ou métalloprotéase), lipase, cutinase, amylase, carbohydrase, cellulase, pectinase, mannanase, arabinase, galactanase, xanthanase, xylanase, DNase, perhydrolase, oxydoréductase (par exemple laccase, peroxydase, peroxygénase et/ou halogénoperoxydase) ; de préférence une protéase, lipase, amylase, cellulase, pectinase, mannanase et/ou DNase.

7. Suspension enzymatique selon l'une quelconque des revendications précédentes, qui comprend en outre un polyol choisi dans le groupe consistant en glycérol, (mono-, di- ou tri-)propylène glycol, alcool de sucre, polypropylène glycol, et/ou polyéthylène glycol, de préférence polyéthylène glycol ou polypropylène glycol ayant un poids moléculaire dans la plage de 200 à 800.

8. Suspension enzymatique selon l'une quelconque des revendications précédentes, dans laquelle la teneur en eau est de 50 à 90 % p/p.

9. Suspension enzymatique selon l'une quelconque des revendications précédentes, qui comprend en outre un inhibiteur enzymatique.

10. Suspension enzymatique selon la revendication 9, dans laquelle l'enzyme est une protéase, et l'inhibiteur est un aldéhyde de peptide ou un dérivé de celui-ci, ou un acide boronique.

11. Suspension enzymatique selon la revendication 9 ou 10, dans laquelle l'inhibiteur de protéase est Cbz-Arg-Ala-Tyr-H, Ac-Gly-Ala-Tyr-H, Cbz-Gly-Ala-Tyr-H, Cbz-Gly-Ala-Tyr-CF3, Cbz-Gly-Ala-Leu-H, Cbz-Val-Ala-Leu-H, Cbz-Val-Ala-Leu-CF3, Moc-Val-Ala-Leu-CF3, Cbz-Gly-Ala-Phe-H, Cbz-Gly-Ala-Phe-CF3, Cbz-Gly-Ala-Val-H, Cbz-Gly-Gly-Tyr-H, Cbz-Gly-Gly-Phe-H, Cbz-Arg-Val-Tyr-H, Cbz-Leu-Val-Tyr-H, Ac-Leu-Gly-Ala-Tyr-H, Ac-Phe-Gly-Ala-Tyr-H, Ac-Tyr-Gly-Ala-Tyr-H, Ac-Phe-Gly-Ala-Leu-H, Ac-Phe-Gly-Ala-Phe-H, Ac-Phe-Gly-Val-Tyr-H, Ac-Phe-Gly-Ala-Met-H, Ac-Trp-Leu-Val-Tyr-H, MeO-CO-Val-Ala-Leu-H, MeNCO-Val-Ala-Leu-H, MeO-CO-Phe-Gly-Ala-Leu-H, MeO-CO-Phe-Gly-Ala-Phe-H, MeSO2-Phe-Gly-Ala-Leu-H, MeSO2-Val-Ala-Leu-H, PhCH2O-P(OH)(O)-Val-Ala-Leu-H, Et-SO2-Phe-Gly-Ala-Leu-H, PhCH2SO2-Val-Ala-Leu-H, PhCH2O-P(OH)(O)-Leu-Ala-Leu-H, PhCH2O-P(OH)(O)-Phe-Ala-Leu-H, ou MeO-P(OH)(O)-Leu-Gly-Ala-Leu-H ou un produit d'addition hydrosulfite de l'un quelconque de ceux-ci, où Cbz est benzyloxycarbonyle et Moc est méthoxycarbonyle ; de préférence où l'inhibiteur de protéase est Cbz-Gly-Ala-Tyr-H ou Moc-Val-Ala-Leu-H, ou un produit d'addition hydrosulfite de ceux-ci, où Cbz est benzyloxycarbonyle et Moc est méthoxycarbonyle.

12. Méthode de préparation d'une composition détergente contenant une enzyme, comprenant l'addition d'une suspension enzymatique selon l'une quelconque des revendications précédentes à une composition comprenant un ou plusieurs ingrédients détergents, tels que des tensioactifs et/ou des adjuvants.

13. Méthode selon la revendication 12, dans laquelle le détergent est un pain de savon ou de détergent.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 450702 A2
- WO 9109943 A
- WO 9422903 A
- WO 8906279 A
- WO 9318140 A
- WO 98020115 A
- WO 0144452 A
- WO 0158275 A
- WO 0158276 A
- WO 03006602 A
- WO 04099401 A
- WO 9219729 A
- WO 96034946 A
- WO 9820115 A
- WO 9820116 A
- WO 99011768 A
- WO 0403186 A
- WO 04041979 A
- WO 07006305 A
- WO 11036263 A
- WO 11036264 A
- WO 2004067737 A
- US 5352604 A
- US 6124127 A
- WO 99027083 A
- WO 99027084 A
- WO 02006442 A
- WO 02092741 A
- WO 03095638 A
- WO 99064619 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 9707202 A
- WO 00060063 A
- WO 2007087508 A
- WO 2009109500 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 2011098531 A
- WO 2013001078 A
- WO 2013001087 A
- WO 2011098579 A
- EP 2013075922 W
- WO 2005056782 A
- WO 2008063400 A
- US 2008145353 A
- US 2007167344 A
- EP 179486 A
- WO 9324618 A
- WO 9510602 A

- WO 9815257 A
- WO 9527046 A
- WO 9704102 A
- WO 0179459 A
- WO 0179458 A
- WO 0179461 A
- WO 0179460 A
- WO 9201046 A
- JP 2238885 A
- WO 9708325 A
- WO 9533836 A
- WO 2008051491 A
- WO 9517413 A
- WO 9522625 A
- US 5223409 A
- WO 9206204 A
- US 4963655 A
- US 5159060 A
- WO 9512655 A
- WO 9529223 A
- WO 9219707 A
- WO 9404653 A
- WO 9404654 A
- US 5442100 A
- US 5488157 A
- US 5472628 A
- WO 9404651 A
- WO 9525791 A
- WO 9813458 A
- WO 9813459 A
- WO 9813460 A
- WO 9813461 A
- WO 9813462 A
- WO 07141736 A
- WO 07145963 A
- WO 09118375 A
- WO 10055052 A
- WO 11036153 A
- WO 2013004636 A
- WO 9847523 A
- US 6500802 B
- US 5436229 A
- WO 2013004635 A
- WO 2008134343 A
- WO 09102854 A
- US 5977053 A
- WO 2006130575 A
- US 5955415 A
- WO 200503274 A
- WO 200503275 A
- WO 200503276 A
- EP 1876226 A
- WO 2007087257 A
- WO 2007087243 A
- WO 2009087523 A
- WO 2007138054 A
- WO 2006108856 A
- WO 2006113314 A
- EP 1867808 A
- WO 2003040279 A
- EP 2169040 A
- US 5275753 A
- WO 9900478 A

**Non-patent literature cited in the description**


- **WHITE** ; **HANDLER** ; **SMITH**. Principles of Biochemistry. McGraw-Hill Book Company, 1973, 271-272
- **SIEZEN et al.** *Protein Engng.*, 1991, vol. 4, 719-737
- **SIEZEN et al.** *Protein Science*, 1997, vol. 6, 501-523
- **DARTOIS et al.** *Biochemica et Biophysica Acta*, 1993, vol. 1131, 253-360
- **H. NEURATH** ; **R.L. HILL**. The Proteins. Academic Press, 1979
- **CUNNINGHAM** ; **WELLS**. *Science*, 1989, vol. 244, 1081-1085
- **HILTON et al.** *J. Biol. Chem.*, 1996, vol. 271, 4699-4708
- **VOS et al.** *Science*, 1992, vol. 255, 306-312
- **SMITH et al.** *J. Mol. Biol.*, 1992, vol. 224, 899-904
- **WLODAVER et al.** *FEBS Lett.*, 1992, vol. 309, 59-64
- **REIDHAAR-OLSON** ; **SAUER**. *Science*, 1988, vol. 241, 53-57
- **BOWIE** ; **SAUER**. *Proc. Natl. Acad. Sci. USA*, 1989, vol. 86, 2152-2156
- **LOWMAN et al.** *Biochemistry*, 1991, vol. 30, 10832-10837
- **DERBYSHIRE et al.** *Gene*, 1986, vol. 46, 145
- **NER et al.** *DNA*, 1988, vol. 7, 127
- **NEEDLEMAN** ; **WUNSCH**. *J. Mol. Biol.*, 1970, vol. 48, 443-453
- **RICE et al.** EMBOSS: The European Molecular Biology Open Software Suite. *Trends Genet*, 2000, vol. 16, 276-277
- *J. Am. Chem. Soc.*, 1978, vol. 100, 1228
- *Org. Synth., Coll.*, vol. 7, 361
- **HODGDON** ; **KALER**. *Current Opinion in Colloid & Interface Science*, 2007, vol. 12, 121-128
- Powdered Detergents. Surfactant science series. Marcel Dekker, Inc., vol. 71